(19) Europäisches Patentamt
European Patent Office
Office européen des brevets

(11) Veröffentlichungsnummer : **0 247 472 B1**

(12) **EUROPÄISCHE PATENTSCHRIFT**

(45) Veröffentlichungstag der Patentschrift : **12.02.92 Patentblatt 92/07**

(21) Anmeldenummer : **87107147.8**

(22) Anmeldetag : **18.05.87**

(51) Int. Cl.⁵ : **C07C 51/235,** C07C 59/10, C07C 59/125, C07C 59/13, C07C 59/68, C07D 319/06, C07D 319/08, // C07D249/08

# (54) Verfahren zur Herstellung von Carbonsäure-Derivaten.

(30) Priorität : **30.05.86 DE 3618114**

(43) Veröffentlichungstag der Anmeldung : **02.12.87 Patentblatt 87/49**

(45) Bekanntmachung des Hinweises auf die Patenterteilung : **12.02.92 Patentblatt 92/07**

(84) Benannte Vertragsstaaten : **BE CH DE FR GB IT LI NL**

(56) Entgegenhaltungen :
**EP-A- 0 011 793**
**DE-A- 1 900 202**
**DE-A- 3 135 946**

(56) Entgegenhaltungen :
**US-A- 3 342 858**
**US-A- 3 506 686**
**PATENT ABSTRACTS OF JAPAN, Band 2, Nr. 8, 20.1.1978, S. 3814C77; & JP-A-52 111514**
**CHEM. BER., 95, 107, 1962**

(73) Patentinhaber : **BAYER AG**
**W-5090 Leverkusen 1 Bayerwerk (DE)**

(72) Erfinder : **Fiege, Helmut, Dr.**
**Walter-Flex-Strasse 23**
**W-5090 Leverkusen 1 (DE)**
Erfinder : **Jautelat, Manfred, Dr.**
**Müllersbaum 28**
**W-5093 Burscheid (DE)**
Erfinder : **Arlt, Dieter, Prof. Dr.**
**Rybnikerstrasse 2**
**W-5000 Koeln 80 (DE)**

EP 0 247 472 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die Erfindung betrifft ein neues Verfahren zur Herstellung von teilweise bekannten Carbonsäure-Derivaten, die als Zwischenprodukte zur Synthese von Kunststoffen, Riechstoffen und von Stoffen mit fungizider bzw. herbizider Wirksamkeit dienen können.

Es ist bereits bekannt, daß sich 2-Phenyl-5-ethyl-1,3-dioxan-5-carbonsäure durch Oxidation von 2-Phenyl-5-ethyl-5-hydroxymethyl-1,3-dioxan mit Kaliumpermaganat in Gegenwart von Aceton herstellen läßt (vgl. Chem. Ber. 95 (1962), 107). Nachteilig an diesem Verfahren ist jedoch, daß es nur mit sehr geringer Selektivität verläuft und die gewünschte Substanz nur in extrem niedriger Ausbeute liefert.

Weiterhin ist bereits beschrieben worden, daß sich 5-Alkyl-1,3-dioxan-5-carbonsäuren ausgehend von 5-Alkyl-5-hydroxymethyl-1,3-dioxanen herstellen lassen (vgl. DE-PS 1 900 202). So geht man bei diesem bekannten Verfahren in der Weise vor, daß man 5-Alkyl-5-hydroxymethyl-1,3-dioxane in flüssiger Phase an Kupfer/Chrom/Barium-Katalysatoren bei Temperaturen zwischen 200 und 350°C dehydriert. Die dabei entstehenden 5-Alkyl-1,3-dioxan-5-carbonsäureester, die das als Ausgangsmaterial eingesetzte 5-Alkyl-5-hydroxymethyl-1,3-dioxan als Alkohol-Komponente enthalten, werden in einem weiteren Reaktionsschritt zu den 5-Alkyl-1,3-dioxan-5-carbonsäuren verseift. Ungünstig an diesem Verfahren ist, daß es sich um eine mehrstufige Synthese handelt und die gewünschten 5-Alkyl-1,3-dioxan-5-carbonsäuren nur in geringer Ausbeute und im Gemisch mit anderen Produkten erhältlich sind. Beeinträchtigend ist außerdem daß sich 5-Alkyl-5-hydroxymethyl-1,3-dioxane, die in 2-Stellung durch Alkylgrupen mono- oder di-substituiert sind, nach dieser Methode nicht zu den entsprechenden Säuren bzw. Estern dehydrieren lassen.

Ferner ist bekannt, daß sich bestimmte 5-Alkyl-5-aceto-1,3-dioxane mit Kaliumpermanganat oder Natriumhypochlorit in alkalischem Medium zu 5-Alkyl-1,3-dioxan-5-carbonsäuren oxidieren lassen (vgl. GB-PS 1 167 274). Von diesem Verfahren ist aber nur bekannt, daß es auf die Umsetzung von solchen 5-Alkyl-5-aceto-1,3-dioxanen anwendbar ist, die in der 2-Stellung außer Wasserstoff keine anderen Substituenten tragen. Darüber hinaus erfordert das Verfahren den Einsatz relativ kostspieliger, großer Mengen an Oxidationsmittel und Alkali, und die dementsprechend anfallenden Salze stellen eine unerwünschte Belastung der Umwelt dar. Schließlich ist bei der Oxidation einer Acetogruppe zu einer Carboxylgruppe der Zugewinn im Molekulargewicht (43 → 45) deutlich geringer als bei der Oxidation einer Hydroxymethylgruppe zu einer Carboxylgruppe (31 → 45). Dieses Verfahren ist also auch aus wirtschaftlichen Gründen nicht befriedigend.

Relativ milde Oxidationsbedingungen, z.B. Sauerstoff in Gegenwart von Katalysatoren, führen bei bekannten Verfahren nur dann zum Erfolg, wenn eine an ein primäres C-Atom gebundene $CH_2$-OH Gruppe oxidiert werden soll (siehe US-PS 3 342 858 und DE-OS 3 135 946).

Im übrigen sind die bisher bekannten 1,3-Dioxan-5-carbonsäuren, die in der 2-Stellung unsubstituiert sind, selbst unter drastischen Bedingungen nur schwierig zu den entsprechenden 2,2-Bis-hydroxymethylalkancarbonsäuren hydrolysierbar lassen (vgl. GB-PS 1 167 274). So werden im Falle der 5-Methyl-1,3-dioxan-5-carbonsäure und der 5-Ethyl-1,3-dioxan-5-carbonsäure selbst dann nur Ausbeuten von unter 40 % der Theorie erreicht, wenn man mit Schwefelsäure als Katalysator und unter Zusatz von Methanol arbeitet, um den entsprechenden Formaldehyd ständig als Methylal aus dem siedenden Reaktionsgemisch zu entfernen.

Es wurde nun gefunden, daß sich Carbonsäure-Derivate der Formel

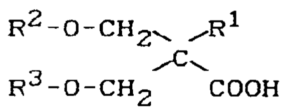

(I)

in welcher

$R^1$ für Wasserstoff, Alkyl, Cycloalkyl oder gegebenenfalls substituiertes Phenyl steht und

$R^2$ und $R^3$ für Wasserstoff stehen oder

$R^2$ und $R^3$ gemeinsam für die Gruppierung der Formel

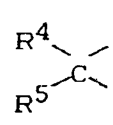

stehen, worin

$R^4$ und $R^5$ unabhängig voneinander für Wasserstoff, Alkyl, Cycloalkyl oder gegebenenfalls substituiertes Phenyl stehen oder

$R^4$ und $R^5$ gemeinsam für eine Alkylenkette stehen, herstellen lassen, indem man 5-Hydroxymethyl-1,3-dioxane der

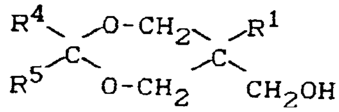

(II)

in welcher

$R^1$, $R^4$ und $R^5$ die oben angegebene Bedeutung haben, in wäßrig-alkalischem Medium mit Sauerstoff oder Sauerstoff enthaltenden Gasen bei Temperaturen zwischen 0°C und dem Siedepunkt des Reaktionsgemisches an einem Palladium-und/oder Platin-Katalysator, gegebenenfalls in Gegenwart eines Aktivators umsetzt und die so erhaltenen Salze mit Kationenaustauschern oder Säuren behandelt und gegebenenfalls anschließend die dabei entstehenden 1,3-Dioxan-5-carbonsäuren der Formel

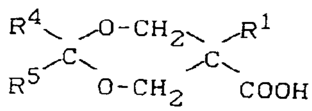

(Ia)

in welcher

$R^1$, $R^4$ und $R^5$ die oben angegebene Bedeutung haben, oder deren Salze mit Wasser, gegebenenfalls in Gegenwart eines Katalysators sowie gegebenenfalls in Gegenwart eines zusätzlichen Verdünnungsmittels bei Temperaturen zwischen 0°C und dem Siedepunkt des Reaktionsgemisches umsetzt.

Der Verlauf des erfindungsgemäßen Verfahrens ist als äußerst überraschend zu bezeichnen, denn im Hinblick auf den bekannten Stand der Technik konnte nicht davon ausgegangen werden, daß sich bei der Oxidation von 5-Hydroxymethyl-1,3-dioxanen direkt 1,3-Dioxan-5-carbonsäuren der Formel (Ia) in extrem hoher Ausbeute herstellen lassen würden. Überraschend ist auch, daß sich die 1,3-Dioxan-5-carbonsäuren der Formel (Ia) glatt zu den entsprechenden 2,2-Bis-hydroxymethylalkancarbonsäuren umsetzen lassen. Es war nämlich zu erwarten, daß die 1,3-Dioxan-5-carbonsäuren der Formel (Ia) ebenso wie die bekannten, in 2-Stellung unsubstituierten 1,3-Dioxan-5-carbonsäuren nur unter drastischen Bedingungen hydrolysieren.

Das erfindungsgemäße Verfahren zeichnet sich durch eine Reihe von Vorteilen aus. So sind die benötigten Ausgangsstoffe einfach und auch in größeren Mengen zugänglich. Weiterhin sind auch die erforderlichen Oxidationsmittel und übrigen Reaktionskomponenten preisgünstig und problemlos zu handhaben. Besonders vorteilhaft ist, daß die gewünschten Produkte in sehr hoher Ausbeute und hervorragender Reinheit anfallen. Außerdem ist das erfindungsgemäße Verfahren breit anwendbar und es lassen sich 2,2-Bis-hydroxymethylalkancarbonsäuren herstellen, die sonst nur auf sehr unwirtschaftliche Weise oder gar nicht zugänglich sind. Im übrigen ist auch die Aufarbeitung der nach der Umsetzung vorliegenden Reaktionsgemische relativ leicht möglich.

Verwendet man 2,2,5-Trimethyl-5-hydroxymethyl-1,3-dioxan als Ausgangsstoff, Sauerstoff als Oxidationsmittel, Palladium auf Aktivkohle als Katalysator, Bisumt-nitrat als Aktivator, wäßrige Natronlauge als Reaktionsmedium und verdünnte wäßrige Schwefelsäure zum Ansäuern, so kann der Verlauf des erfindungsgemäßen Verfahrens durch das folgende Formelschema veranschaulicht werden:

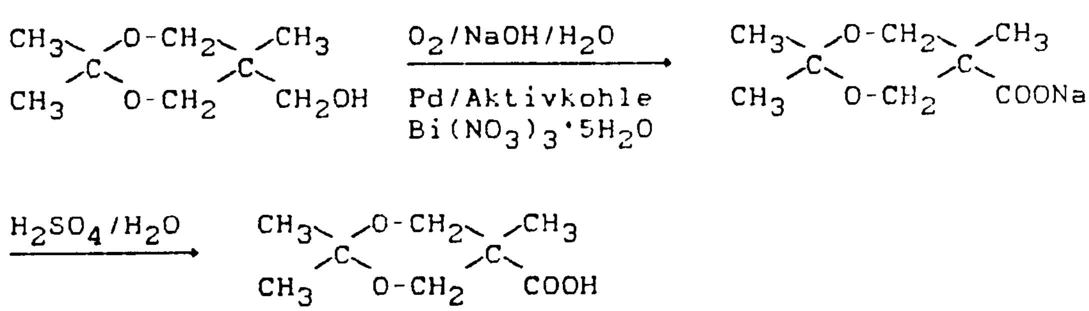


Erhitzt man 2,2,5-Trimethyl-1,3-dioxan-5-carbonsäure mit Wasser auf 95 bis 102°C, so kann der Verlauf der Hydrolyse durch das folgende Formelschema veranschaulicht werden:

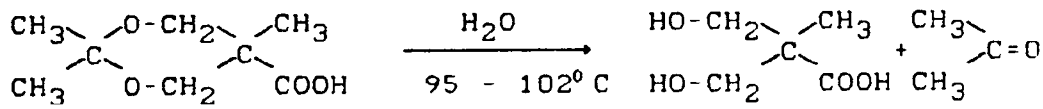


Die bei dem erfindungsgemäßen Verfahren als Ausgangsstoffe benötigten 5-Hydroxymethyl-1,3-dioxane sind durch die Formel (II) allgemein definiert. In dieser formel stehen $R^1$, $R^4$ und $R^5$ unabhängig voneinander vorzugsweise für Wasserstoff, geradkettiges oder verzweigtes Alkyl mit 1 bis 12 Kohlenstoffatomen, Cycloalkyl mit 3 bis 8 Kohlenstoffatomen oder für gegebenenfalls durch Halogen und/oder Alkyl mit 1 bis 6 Kohlenstoffatomen substituiertes Phenyl. Außerdem stehen $R^4$ und $R^5$ auch gemeinsam für eine Alkylenkette mit vorzugsweise 4 bis 6 Kohlenstoffatomen.

Besonders bevorzugt sind diejenigen Stoffe der Formel (II), in denen $R^1$, $R^4$ und $R^5$ unabhängig voneinander für Wasserstoff, geradkettiges oder verzweigtes Alkyl mit 1 bis 8 Kohlenstoffatomen, insbesondere mit 1 bis 6 Kohlenstoffatomen, Cycloalkyl mit 3 bis 6 Kohlenstoffatomen oder für gegebenenfalls durch Fluor, Chlor, Brom und/oder Alkyl mit 1 bis 4 Kohlenstoffatomen substituiertes Phenyl stehen. Besonders bevorzugt sind auch diejenigen Stoffe der Formel (II), in denen $R^4$ und $R^5$ gemeinsam für eine Alkylenkette mit 4 oder 5 Kohlenstoffatomen stehen.

Als spezielle Reste seien für die Substituenten $R^1$, $R^4$ und $R^5$ genannt: Wasserstoff, Methyl, Ethyl, Propyl, Isopropyl, Butyl, Isobutyl, sec-Butyl, tert.-Butyl, Pentyl, Isopentyl, tert.-Amyl, Neopentyl, Cyclopropyl, Isohexyl, Cyclohexyl, Heptyl, Isoheptyl, tert.-Octyl, Isooctyl, Nonyl, Isononyl, Decyl, Isodecyl, Undecyl, Isoundecyl, Dodecyl, Isododecyl, Phenyl und 4-Methyl-phenyl. Die Substituenten $R^4$ und $R^5$ können auch miteinander verknüpft sein und für eine Alkylenkette mit 4 oder 5 Kohlenstoffatomen stehen.

Als Beispiele für 5-Hydroxymethyl-1,3-dioxane der Formel (II) seien genannt:
5-Hydroxymethyl-1,3-dioxan, 2-Methyl-5-hydroxymethyl-1,3-dioxan, 2,2-Dimethyl-5-hydroxymethyl-1,3-dioxan, 5-Methyl-5-hydroxymethyl-1,3-dioxan, 2,5-Dimethyl-5-hydroxymethyl-1,3-dioxan, 2-Ethyl-5-methyl -5-hydroxymethyl-1,3-dioxan, 2-Propyl-5-methyl -5-hydroxymethyl-1,3-dioxan, 2-Isopropyl-5-methyl -5-hydroxymethyl-1,3-dioxan, 2-Butyl-5-methyl -5-hydroxymethyl-1,3-dioxan, 2,2,5-Trimethyl -5-hydroxymethyl-1,3-dioxan, 2,5-Dimethyl-2-ethyl -5-hydroxymethyl-1,3-dioxan, 2,2-Diethyl-5-methyl-5-hydroxymethyl-1,3-dioxan, 2,5-Dimethyl-2-isopropyl-5-hydroxymethyl-1,3-dioxan, 2,5-Dimethyl-2-isobutyl-5-hydroxymethyl-1,3-dioxan, 5-Ethyl-5-hydroxymethyl-1,3-dioxan, 2-Methyl-5-ethyl-5-hydroxymethyl-1,3-dioxan, 2,5-Diethyl -5-hydroxymethyl-1,3-dioxan, 2-Propyl-5-ethyl-5-hydroxymethyl-1,3-dioxan, 2-Butyl-5-ethyl-5-hydroxymethyl-1,3-dioxan, 2,2-Dimethyl-5-ethyl-5-hydroxymethyl-1,3-dioxan, 2-Methyl-2,5-diethyl-5-hydroxymethyl-1,3-dioxan, 2,2,5-Triethyl-5-hydroxymethyl-1,3-dioxan, 2-Methyl-2-isopropyl-5-ethyl-5-hydroxymethyl-1,3-dioxan, 2-Methyl-2-isobutyl-5-ethyl-5-hydroxymethyl-1,3-dioxan, 5-Propyl-5-hydroxymethyl-1,3-dioxan, 2,2-Dimethyl-5-propyl-5-hydroxymethyl-1,3-dioxan, 5-Isopropyl-5-hydroxymethyl-1,3-dioxan, 2,2-Dimethyl-5-isopropyl-5-hydroxymethyl-1,3-dioxan, 5-n-Butyl-5-hydroxymethyl-1,3-dioxan, 2,2-Dimethyl-5-n-butyl-5-hydroxymethyl-1,3-dioxan, 5-Isobutyl-5-hydroxymethyl-1,3-dioxan, 2,2-Dimethyl-5-isobutyl-5-hydroxymethyl-1,3-dioxan.

Die 5-Hydroxymethyl-1,3-dioxane der Formel (II) sind bekannt oder lassen sich nach bekannten Methoden in einfacher Weise herstellen (vgl. J. prakt. Chem. 4. Reihe, Bd. 6 (1967) 170-179 und J. Org. Chem. 26 (1961), 3571-3574). So können 5-Hydroxymethyl-1,3-dioxane der Formel (II) zum Beispiel durch Umsetzung von 1,1,1-Trishydroxymethylalkanen mit Aldehyden oder Ketonen erhalten werden.

Bei der Durchführung des erfindungsgemäßen Verfahrens kommen als Katalysatoren alle üblichen Palladium- und Platin-Katalysatoren sowie deren Gemische in Betracht. Die Katalysatoren können zusätzlich noch mit Aktivatoren oder Gemischen verschiedener Aktivatoren kombiniert werden. Als Aktivatoren kommen hierbei vorzugsweise Blei, Bismut, Bleiverbindungen und Bismutverbindungen sowie deren Gemische infrage.

Bei der Durchführung des erfindungsgemäßen Verfahrens können das als Katalysator zu verwendende Platin oder Palladium oder Gemische, die diese Metalle enthalten, in üblicher Weise eingesetzt werden. So können die Stoffe in elementarer Form, z.B. als sogenanntes Platinoder Palladium-Mohr, gegebenenfalls in Kombination mit anderen Platinmetallen, oder auch in Form von Verbindungen, wie z.B. den Oxiden, zugegeben werden.

Das Platin oder das Palladium können auch auf einem Träger aufgebracht sein. Als Träger geeignet sind beispielsweise Aktivkohle, Graphit, Kieselgur, Kieselgel, Spinelle, Aluminiumoxid, Asbest, Calciumcarbonat, Bariumsulfat oder auch organische Trägermaterialien.

Bevorzugt werden als Trägermaterial Aktivkohlen, beispielsweise sogenannte Medizinalkohlen oder aus Holz hergestellte Aktivkohlen, wie sie vielfach für Entfärbungszwecke verwendet werden, eingesetzt.

Der Platin- und/oder Palladium-Gehalt der Trägerkatalysatoren kann innerhalb eines größeren Bereiches variiert werden. Im allgemeinen verwendet man Trägerkatalysatoren, in denen der Gehalt an diesen Metallen zwischen 0,01 und 20 Gew.-%-, vorzugsweise zwischen 0,1 und 15 Gew.-%- liegt.

Auch die Mengen, in denen die Platin- und/oder Palladium-Katalysatoren eingesetzt werden, können in einem größeren Bereich variiert werden. Die Mengen hängen unter anderem von der gewünschten Oxidationsgeschwindigkeit ab. Im allgemeinen wählt man die Katalysatormenge so, daß zwischen 0,01 und 20 g, vorzugsweise zwischen 0,05 und 10 g Platin und/oder Palladium pro Mol 5-Hydroxymethyl-1,3-dioxan der Formel (II) im Reaktionsgemisch anwesend sind.

Bei der Durchführung des erfindungsgemäßen Verfahrens ist es auch möglich, Platin in Kombination mit Palladium als Katalysator einzusetzen.

Die Aktivität und/oder Selektivität der Platinkatalysatoren wird bei dem erfindungsgemäßen Verfahren durch die Gegenwart von Blei und/oder Bismut und/oder deren Verbindungen als Aktivator erheblich gesteigert.

Palladiumkatalysatoren weisen auch ohne Zusatz der vorgenannten Aktivatoren schon eine so überraschend hohe Aktivität und Selektivität auf, daß bei ihnen gegebenenfalls auf den Zusatz der vorgenannten Aktivatoren verzichtet werden kann.

Der Zusatz der vorgenannten Aktivatoren wirkt sich auch positiv auf die Wiederverwendbarkeit der Katalysatoren aus.

Die Mengen, in denen die Aktivatoren bei der Durchführung des erfindungsgemäßen Verfahrens gegebenenfalls eingesetzt werden, können in einem größeren Bereich variiert werden. Die Aktivatorwirkung macht sich bereits bei Zusätzen von 5 x $10^{-6}$ Mol Metall oder Metallverbindung pro Mol 5-Hydroxymethyl-1,3-dioxan bemerkbar. Es können auch 0,1 Mol oder mehr Aktivator pro Mol 5-Hydroxymethyl-1,3-dioxan eingesetzt werden, jedoch bieten diese hohen Zusätze im allgemeinen keinen Vorteil. Üblicherweise werden die Aktivatoren in Mengen von etwa 1 x $10^{-5}$ bis 1 x $10^{-1}$ Mol, vorzugsweise 2 x $10^{-5}$ bis 2 x $10^{-2}$ Mol, pro Mol zu oxidierendem 5-Hydroxymethyl-1,3-dioxan zugegeben.

Die bei der Durchführung des erfindungsgemäßen Verfahrens gegebenenfalls als Aktivatoren zu verwendenden Metalle können in elementarer Form und/oder in Form ihrer Verbindungen, z.B. als Oxide, Hydroxide, Oxidhydrate oder Sauerstoffsäuren oder als Salze von Wasserstoffsäuren, wie Chloride, Bromide, Jodide, Sulfide, Selenide, Telluride, oder als Salze von anorganischen Sauerstoffsäuren, wie Nitrate, Nitrite, Phosphite, Phosphate, Arsenite, Arsenate, Antimonite, Antimonate, Bismutate, Stannate, Plumbate, Selenite, Selenate, Tellurite, Tellurate, Borate oder als Salze von Sauerstoffsäuren, die von Übergangsmetallen abstammen, wie Vanadate, Niobate, Tantalate, Chromate, Molybdate, Wolframate, Permanganate, oder als Salze organischer aliphatischer oder aromatischer Säuren, wie Formiate, Acetate, Propionate, Benzoate, Salicylate, Lactate, Mandelate, Glyoxylate, Alkoxyacetate, Citrate, Phenolate, oder als Komplexverbindungen oder als metallorganische Verbindung eingesetzt werden.

Die Aktivatoren können im Reaktionsgemisch jeweils löslich, teilweise löslich oder unlöslich sein.

Es ist auch möglich, die Aktivatoren in Kombination mit anderen nicht als Aktivator beanspruchten Elementen oder Verbindungen in das erfindungsgemäße Verfahren einzusetzen.

Die bei der Durchführung des erfindungsgemäßen Verfahrens gegebenenfalls einzusetzenden Aktivatoren können in unterschiedlichen und auch in gemischten Wertigkeitsstufen vorliegen. Es können auch Änderungen in der Wertigkeit während der Reaktion eintreten. Sofern die Aktivatoren nicht bereits als Oxide und/oder Hydroxide zugegeben werden, ist es möglich, daß sie sich im alkalischen Medium ganz oder teilweise in diese umwandeln. Nach der Reaktion kann der Platin und/oder Palladiumkatalysator mit dem Aktivator (soweit dieser ungelöst geblieben ist) abfiltriert und für weitere Oxidationsreaktionen verwendet werden. Verluste an Platinbzw. Palladiumkatalysator und/oder an Aktivator sind gegebenenfalls zu ersetzen.

Der Aktivator kann als Feststoff, vorzugsweise in feinverteilter Form, oder in gelöster Form den Reaktionskomponenten zugesetzt werden. Man kann den Aktivator auch schon bei der Herstellung des Platin- bzw. Palladium-Katalysators zugeben oder den Platin- bzw. Palladiumkatalysator mit dem Aktivator imprägnieren. Der Aktivator kann auch als Trägermaterial für das Platinmetall dienen.

Die Oxidation nach dem erfindungsgemäßen Verfahren erfolgt in wäßrig-alkalischem Medium bei einem pH-Wert >7. Die Einstellung des geeigneten pH-Wertes erfolgt durch Zugabe von Alkalien. Als Alkalien kommen Verbindungen der Alkaliund/oder Erdalkalimetalle in Betracht, wie die Hydroxide, Carbonate, Hydrogencarbonate, Phosphate und Borate. Bevorzugt werden die Hydroxide und/oder Carbonate des Natriums und/oder Kaliums als Alkali eingesetzt.

Da bei dem erfindungsgemäßen Verfahren pro Mol gebildeter Säure 1 Mol Alkali ($OH^{\ominus}$) verbraucht wird, liegt die einzusetzende Alkalimenge bei etwa 1 Mol Alkali pro Mol 5-Hydroxymethyl-1,3-dioxan. Im allgemeinen werden etwa 1 bis 1,5 Mol Alkali pro Mol 5-Hydroxymethyl-1,3-dioxan eingesetzt.

Höhere Verhältnisse können angewendet werden, bringen jedoch gewöhnlich keine wesentlichen Vorteile mehr. Wenn es erwünscht ist, daß vom eingesetzten 5-Hydroxymethyl-1,3-dioxan nur ein Teil zur 1,3-Dioxan-5-carbonsäure oxidiert wird, kann auch entsprechend weniger Alkali eingesetzt werden.

Das Alkali kann zu Beginn auf einmal oder auch erst im Verlauf der Reaktion intermittierend oder kontinuierlich dem Reaktionsgemisch hinzugefügt werden.

Die 5-Hydroxymethyl-1,3-dioxane werden bevorzugt in wäßriger Lösung oxidiert. Es können aber auch noch andere inerte organische Substanzen, beispielsweise Lösungs-mittel, wie tert.-Butanol, Aceton, Dioxan und/oder Toluol und/oder Nebenprodukte aus der Herstellung des 5-Hydroxymethyl-1,3-dioxans anwesend sein. Die 5-Hydroxymethyl-1,3-dioxane werden im allgemeinen in Form einer 2 bis 40 %igen Lösung eingesetzt. Welche Konzentration zweckmäßig ist, hängt unter anderem von der gewünschten Reaktionsgeschwindigkeit ab. Letztere nimmt bei höheren 5-Hydroxymethyl-1,3-dioxan-Konzentrationen allmählich ab. Es ist auch möglich, Gemische verschiedener 5-Hydroxymethyl-1,3-dioxane zu oxidieren.

Die Reaktionstemperaturen können bei der Durchführung des erfindungsgemäßen Verfahrens innerhalb eines größeren Bereiches variiert werden. So kann die Reaktionstemperatur zwischen dem Erstarrungspunkt und dem Siedepunkt des Reaktionsgemisches liegen. Die im Einzelfall anzuwendende Reaktionstemperatur richtet sich unter anderem nach dem Katalysatorsystem, der Katalysatormenge, der Alkalikonzentration, den Substanzeigenschaften der Edukte und Produkte und den technischen Gegebenheiten, wie zum Beispiel gewünschte Reaktionsgeschwindigkeit oder Wärmeabfuhr. Im allgemeinen arbeitet man bei Temperaturen zwischen 0°C und dem Siedepunkt des Reaktionsgemisches, vorzugsweise zwischen 40°C und 100°C.

Die Reihenfolge, in der der Platin- und/oder Palladium-Katalysator sowie gegebenenfalls Aktivator, wäßriges Alkali und 5-Hydroxymethyl-1,3-dioxan zusammengegeben werden, ist beliebig. So können der Platin- und/oder Palladium-Katalysator sowie gegebenenfalls Aktivator der Mischung oder Lösung aus wäßrigem Alkali und 5-Hydroxymethyl-1,3-dioxan zugesetzt werden. Man kann auch Platin- und/oder Palladium-Katalysator sowie gegebenenfalls Aktivator vorlegen und die Mischung aus wäßrigem Alkali und 5-Hydroxymethyl-1,3-dioxan zusetzen. Schließlich ist es auch möglich, den Platin- und/oder Palladium-Katalysator, einen Teil des wäßrigen Alkalis sowie gegebenenfalls Aktivator vorzulegen und 5-Hydroxymethyl-1,3-dioxan dann zusammen mit dem restlichen Alkali hinzuzufügen. Ferner ist es möglich, den Aktivator der Mischung der übrigen Komponenten zuzusetzen.

Im allgemeinen wird das erfindungsgemäße Verfahren so durchgeführt, daß man Sauerstoff oder Sauerstoff enthaltende Gase, wie Luft, mit dem Reaktionsgemisch, das wäßriges Alkali, den Platin- und/oder Palladium-Katalysator, gegebenenfalls Aktivator sowie 5-Hydroxymethyl-1,3-dioxan enthält, in intensiven Kontakt bringt. Der Katalysator braucht im Reaktionsgemisch nicht als Pulver suspendiert vorzuliegen, sondern kann auch in gekörnter Form als Festbett angeordnet sein, das von den übrigen Komponenten durchströmt wird.

Der Druck kann bei der Durchführung des erfindungsgemäßen Verfahrens innerhalb eines größeren Bereiches variiert werden. Im allgemeinen arbeitet man unter Drucken zwischen 0,5 und 10 bar. Vorzugsweise führt man die Oxidation bei Atmosphärendruck durch.

Der Verlauf der Umsetzung kann durch Messung der aufgenommenen Sauerstoffmenge verfolgt werden. Die Umsetzung wird abgebrochen, wenn die für die Herstellung der jeweiligen 1,3-Dioxan-5-carbonsäure theoretisch erforderliche Sauerstoffmenge aufgenommen worden ist. Im allgemeinen hört die Sauerstoffaufnahme in diesem Stadium von selbst auf, oder sie verlangsamt sich deutlich.

Nach der Durchführung der erfindungsgemäßen Oxidation erfolgt die Weiterverarbeitung des Reaktionsgemisches nach üblichen Methoden. Im allgemeinen geht man so vor, daß man den Katalysator sowie gegebenenfalls vorhandenen ungelösten Aktivator zum Beispiel durch Filtration abtrennt. Die erhaltenen Alkalisalzlösungen der 1,3-Dioxan-5-carbonsäuren können, gegebenenfalls nach vorherigem Eindampfen, als solche weiterverwendet werden. Man kann die Alkalisalzlösungen der 1,3-Dioxan-5-carbonsäuren auch vollständig, also bis zur Trockne, eindampfen und den verbleibenden Salzrückstand weiterverwenden. Wenn die freien 1,3-Dioxan-5-carbonsäuren hergestellt werden sollen, geht man im allgemeinen so vor, daß man das verbleibende Reaktionsgemisch, gegebenenfalls nach vorherigem Einengen unter vermindertem Druck, mit verdünnter Mineralsäure ansäuert, dann mit einem in Wasser wenig löslichen organischen Solvens extrahiert und die organische Phase, gegebenenfalls nach vorherigem Trocknen, einengt. Als Mineralsäuren können hierbei vorzugsweise Salzsäure, Schwefelsäure oder Phosphorsäure eingesetzt werden. Als organische Solventien für die Extraktion kommen vorzugsweise Ether, wie Diethylether und Diisopropylether, weiterhin Ketone, wie Methylisobutylketon, und außerdem gegebenenfalls halogenierte aliphatische oder aromatische Kohlenwasserstoffe, wie Methylenchlorid, Chloroform, Tetrachlormethan oder Toluol, in Betracht. Die freie Säure wird dann durch Eindampfen des Extraktes gewonnen, wobei das Abziehen des Verdünnungsmittels auch unter vermindertem Druck geschehen kann.

Es ist auch möglich, die jeweilige 1,3-Dioxan-5-carbonsäure aus den zunächst anfallenden wäßrigen Alkalisalz-Lösungen an einem Kationenaustauscher freizusetzen und durch schonendes Eindampfen der salzfreien, wäßrigen Lösung zu isolieren. Bei unvollständigem Umsatz des verwendeten 5-Hydroxymethyl-1,3-dioxans kann man dieses vor dem Ansäuern durch Extraktion der wäßrigen Alkalisalz-Lösung mit einem in Wasser wenig löslichen organischen Solvens entfernen, wiedergewinnen und gegebenenfalls erneut als Ausgangsmaterial einsetzen.

Die bei dem erfindungsgemäßen Verfahren zunächst anfallenden 1,3-Dioxan-5-carbonsäuren der Formel

(Ia) lassen sich - sofern dieses gewünscht wird - zu den entsprechen den 2,2-Bis-hydroxymethyl-alkancarbonsäuren hydrolysieren. Für diese Hydrolyse können die 1,3-Dioxan-5-carbonsäuren der Formel (Ia) oder auch deren Salze eingesetzt werden. Als Salze können hierbei vorzugsweise Alkalimetall- oder Erdalkalimetall-Salze, wie Natrium-, Kalium-, Magnesium-oder Calcium-Salze, eingesetzt werden. Die Salze oder 1,3-Dioxan-5-carbonsäuren der Formel (Ia) lassen sich nach üblichen Salzbildungsreaktionen herstellen. Sie fallen bei der Synthese der 1,3-Dioxan-5-carbonsäuren der Formel (Ia) nach dem erfindungsgemäßen Verfahren an und können gegebenenfalls nach vorheriger Isolierung für die Weiterreaktion verwendet werden.

Die Hydrolyse erfolgt bei dem erfindungsgemäßen Verfahren mit Wasser, gegebenenfalls in Gegenwart eines Katalysators. Als Katalysatoren kommen hierbei alle für derartige Umsetzungen üblichen Reaktionsbeschleuniger infrage. Vorzugsweise verwendbar sind Säuren, wie zum Beispiel Schwefelsäure oder Salzsäure.

Die Reaktionstemperaturen können bei der Durchführung der Hydrolyse innerhalb eines größeren Bereiches variiert werden. Im allgemeinen arbeitet man bei Temperaturen zwischen 0°C und dem Siedepunkt des Reaktionsgemisches.

Als zusätzliche Verdünnungsmittel können bei der Durchführung der Hydrolyse alle üblichen inerten organischen Solventien sowie auch Alkohole eingesetzt werden.

Bei der Durchführung der Hydrolyse geht man im allgemeinen so vor, daß man die 1,3-Dioxan-5-carbonsäuren der Formel (Ia) oder deren Salze mit Wasser, gegebenenfalls unter Zugabe einer katalytischen Menge an Säure, erhitzt.

Ist bei der Durchführung des erfindungsgemäßen Verfahrens ausschließlich die Synthese von 2,2-Bis-hydroxymethylalkancarbonsäuren gewünscht, so kann auf eine vorherige Isolierung der entsprechenden 1,3-Dioxan-5-carbonsäuren verzichtet werden. In diesem Fall genügt es, die bei der Oxidation erhaltene Alkalisalz-Lösung der jeweiligen 1,3-Dioxan-5-carbonsäure anzusäuern und zu erhitzen. Die dabei abspaltende Carbonylverbindung kann, gegebenenfalls zusammen mit Wasser, abdestilliert oder - bei geringer Wasserlöslichkeit - auch abdekantiert werden. Besonders gut geeignet zur Darstellung von 2,2-Bis-hydroxymethyl-alkancarbonsäuren sind 2,2-Dimethyl-1,3-dioxan-5-carbonsäuren, da sie leicht spaltbar sind und das freiwerdende Aceton gut abdestillierbar und wiedergewinnbar ist.

Falls die wie vorstehend erhaltenen salzhaltigen, wäßrigen Lösungen der 2,2-Bis-hydroxymethyl-alkancarbonsäuren nicht schon als solche verwendet werden, kann eine Isolierung der 2,2-Bis-hydroxymethyl-alkancarbonsäuren z.B. durch Extraktion mit polaren Lösungsmitteln wie n-Butanol, Cyclohexanol, Cyclohexanon, Ethylacetat, Octanol, Methylisobutylketon usw. erfolgen. Als Extraktionsmittel kann auch die bei der Spaltung der 1,3-Dioxan-5-carbonsäure freiwerdende Carbonylverbindung verwendet oder mitverwendet werden. Man kann die Lösungen auch zur Trockne eindampfen und die 2,2-Bis-hydroxymethyl-alkancarbonsäure aus dem salzhaltigen Rückstand mit polaren organischen Lösungsmitteln herauslösen. In vielen Fällen ist eine Reindarstellung der 2,2-Bis-hydroxymethyl-alkancarbonsäuren nicht erforderlich; es kann vielmehr bereits der salzhaltige Eindampfrückstand verwendet werden. Zur Gewinnung der salzfreien 2,2-Bis-hydroxymethyl-alkancarbonsäuren kann man auch die wäßrige Alkalisalzlösung der jeweiligen 1,3-Dioxan-5-carbonsäure nach der Oxidation an einem Kationenaustauscher umsetzen und dann unter Abdestillation des Wassers und der abgespaltenen Carbonylverbindung eindampfen.

Die nach dem erfindungsgemäßen Verfahren herstellbaren 1,3-Dioxan-5-carbonsäuren und 2,2-Bis-hydroxymethyl-alkancarbonsäuren sind wertvolle Zwischenprodukte zur Herstellung von Kunststoffen, Riechstoffen und von Stoffen mit fungizider bzw. herbizider Wirkung.

Die 2,2-Bis-hydroxymethyl-alkancarbonsäuren lassen sich zum Beispiel mit Hilfe von anorganischen Säurechloriden in 2,2-Bis-chlormethyl-alkancarbonsäurechloride überführen, die als Ausgangsmaterialien zur Synthese von herbizid wirksamen Triazinon-Derivaten bzw. zur Herstellung von fungizid wirksamen Triazolyl-Derivaten verwendbar sind.

Es kann z.B. das 2,2′-Bis-chlorpivaloylchlorid, gegebenenfalls nach vorherigem Austausch der Chloratome gegen Fluoratome, durch Umsetzung mit Trimethylsilylcyanid in die entsprechenden Halogen-pivaloylcyanide überführt werden, welche sich nach bekannten Methoden zu 1,2,4-Triazin-5-on-Derivaten umsetzen lassen (vgl. DE-OS 3 037 300).

Ferner kann z.B. das 2,2′-Bis-chlorpivaloylchlorid durch Behandlung mit Kaliumfluorid in das 2,2′-Bis-fluorpivaloylfluorid überführt werden, welches mit Malonsäure-monoethylester-Magnesium-Salz zum 2,2-Bis-fluormethylbutan-3-on reagiert. Letzteres läßt sich mit Brom zu dem 2,2-Bis-fluormethyl-4-brom-butan-3-on umsetzen, das nach Reaktion mit 1,2,4-Triazol das 2,2-Bis-fluormethyl-4-(1,2,4-triazol-1-yl)-butan-3-on ergibt. Dieses läßt sich durch Umsetzung mit Cyclohexylmethylbromid und Reduktion des zunächst entstehenden Produktes mit Natriumborhydrid in das 2,2-Bis-fluormethyl-5-cyclohexyl-4-(1,2,4-triazol-1-yl)-pentan-3-ol überführen (vgl. DE-OS 3 326 875, DE-OS 2 951 163 und JP-OS 61 572 (1985)). Die genannten Umsetzungen lassen sich durch das folgende Formelschema wiedergeben:

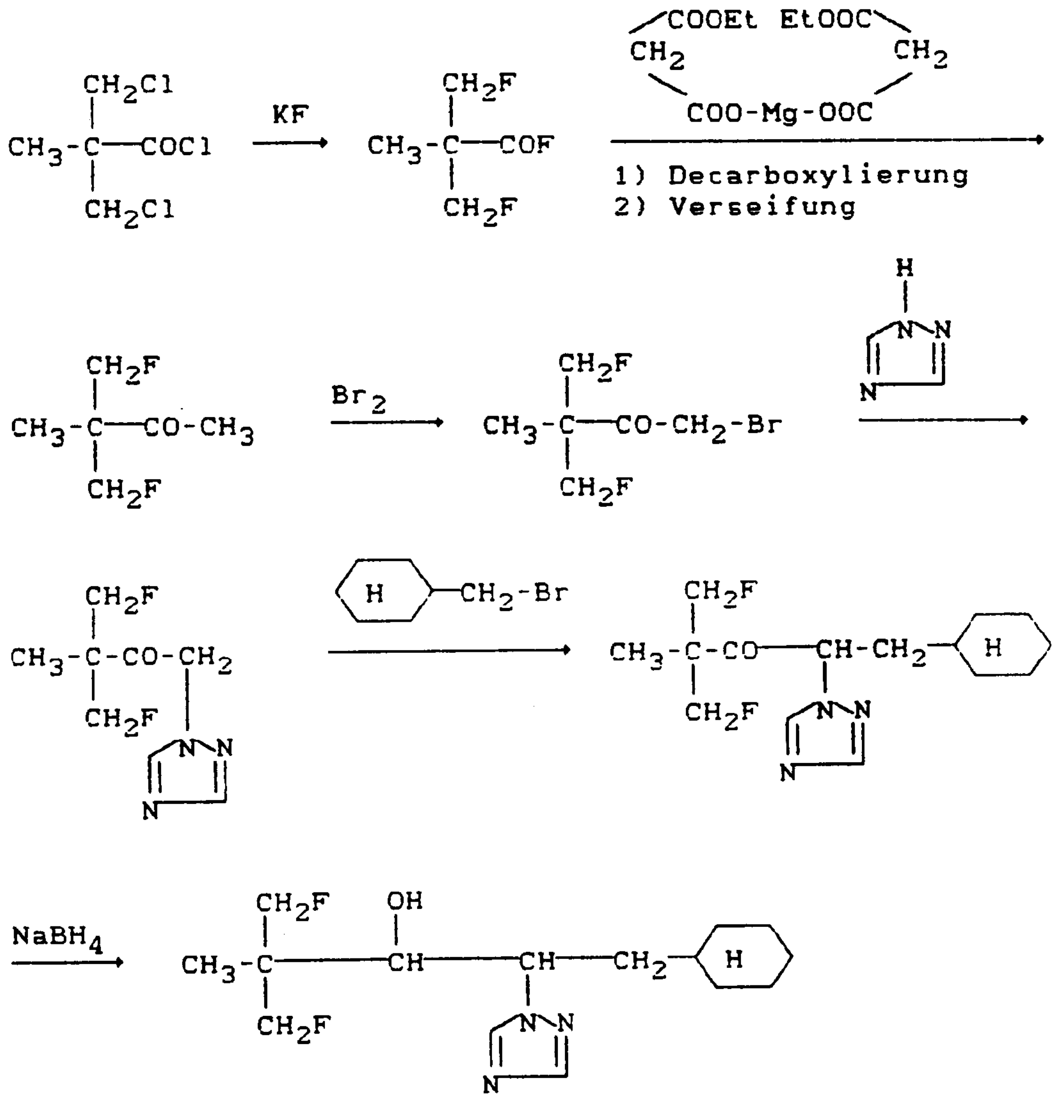


Die Durchführung des erfindungsgemäßen Verfahrens wird durch die folgenden Beispiele veranschaulicht.

Beispiel 1

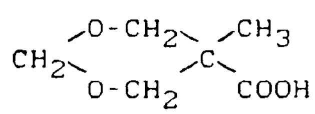

In ein mit Rührer, Innenthermometer und Gaszuleitung versehenes, über einen Heizmantel thermostatisierbares Reaktionsgefäß werden eine Lösung von 13,2 g (0,1 Mol) 5-Methyl-5-hydroxymethyl-1,3-dioxan in 100 ml (0,12 Mol) 1,2 m wäßriger Natronlauge, 1 g Aktivkohlepulver mit 5 Gew.-% Palladiumgehalt und 0,030 g $Bi(NO_3)_3 \cdot 5\ H_2O$ gegeben.

Nach dem Austausch der Luft im Reaktionsgefäß gegen Sauerstoff wird auf 80°C erwärmt und bei dieser Temperatur unter kräftigem Rühren Sauerstoff unter Normaldruck eingeleitet, bis die Reaktion nach 90 Minuten zum Stillstand kommt und 0,1 Mol Sauerstoff aufgenommen sind.

Nach dem Abfiltrieren des Katalysators, Nachwaschen mit 20 ml Wasser und Ansäuern des Filtrats mit 50 %iger Schwefelsäure auf pH 2, wird mit Methylisobutylketon extrahiert. Nach dem Abziehen des Extraktionsmittels unter vermindertem Druck verbleibt ein Produkt, das gemäß Gaschromatogramm zu 99,1 % aus 5-Methyl-1,3-dioxan-5-carbonsäure besteht. Die Ausbeute errechnet sich danach zu 97,7 % der Theorie.
Fp. 86°C (nach Umkristallisation aus Ligroin).

Der abfiltrierte Katalysator kann bei weiteren Ansätzen wiederverwendet werden.

Beispiel 2

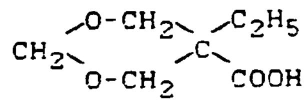

In die im Beispiel 1 beschriebene Apparatur werder. 21,9 g (0,15 Mol) 5-Ethyl-5-hydroxymethyl-1,3-dioxan, 100 ml (0,17 Mol) 1,7 m wäßrige Natronlauge, 1 g Aktivkohlepulver mit 5 Gew.-% Palladiumgehalt und 0,030 g $Bi(NO_3)_3 \cdot 5\ H_2O$ eingebracht. Wie im Beispiel 1 beschrieben, wird mit Sauerstoff unter Normaldruck bei 80°C oxidiert. Nach 3 Stunden sind 0,15 Mol Sauerstoff aufgenommen, und die Reaktion ist praktisch zum Stillstand gekommen.

Nach dem Abfiltrieren des Katalysators, Nachwaschen mit etwas Wasser und Ansäuern des Filtrates mit konzentrierter Salzsäure auf pH 2,5 wird mehrfach mit Ether extrahiert. Nach dem Abziehen des Ethers verbleiben 23,2 g eines Rückstandes mit dem Schmelzpunkt 73 bis 76°C. Der Rückstand enthält 22,15 g 5-Ethyl-1,3-dioxan-5-carbonsäure und 0,85 g 5-Ethyl-5-hydroxymethyl-1,3-dioxan. Der Umsatz beträgt demnach 96,1 %. Danach errechnet sich eine Ausbeute von 92,3 % der Theorie; die Selektivität beträgt 96 %.
Fp. 77 bis 78°C (nach Umkristallisation aus Ligroin).

Beispiel 3

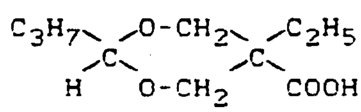

Zu 9,2 g (0,0489 Mol) 2-Propyl-5-ethyl-5-hydroxymethyl-1,3-dioxan-Iosmerengemisch 75:25 werden 100 ml 1 n wäßrige Natronlauge, 1 g Aktivkohlepulver mit 5 Gew.-% Palladiumgehalt und 0,015 g $Bi(NO_3)_3 \cdot 5\ H_2O$ gegeben. Dann wird wie im Beispiel 1 beschrieben bei 90°C unter guter Durchmischung Sauerstoff eingeleitet. Nach 75 Minuten kommt die Sauerstoff-Aufnahme praktisch zum Stillstand, und es sind 0,046 Mol Sauerstoff aufgenommen worden.

Nach dem Abfiltrieren des Katalysators und Nachwaschen mit etwas Wasser wird das alkalische Filtrat mit 4 x 50 ml Methylenchlorid extrahiert. Nach dem Abdestillieren des Methylenchlorids von den vereinigten Extrakten verbleiben 0,6 g Ausgangsmaterial. Die wäßrige Phase wird dann mit 20 %iger Salzsäure auf pH 1,5 angesäuert und erneut mit 4 x 5 ml Methylenchlorid extrahiert. Nach dem Abdestillieren des Methylenchlorids von den vereinigten organischen Phasen verbleiben 9,0 g 2-Propyl-5-ethyl-1,3-dioxan-5-carbonsäure. Laut gaschromatographischer Analyse handelt es sich um ein Isomerengemisch, in dem die beiden Isomeren im Verhältnis 78:22 vorliegen.

Beispiel 4

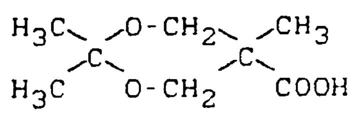

Zu einer Lösung von 16 g (0,1 Mol) 2,2,5-Trimethyl-5-hydroxymethyl-1,3-dioxan in 100 ml (0,12 Mol) 1 n wäßriger Natronlauge werden 1 g 5 % Palladium enthaltendes Aktivkohlepulver und 0,030 g $Bi(NO_3)_3 \cdot 5\ H_2O$ gegeben.

Nach dem Verdrängen der Luft aus dem Reaktionsgefäß mit Sauerstoff wird auf 80°C erwärmt und bei dieser Temperatur unter kräftigem Rühren Sauerstoff unter Normaldruck eingeleitet bis die Sauerstoff-Aufnahme nach 100 Minuten zum Stillstand kommt und 0,1 Mol Sauerstoff aufgenommen sind.

Nach dem Abfiltrieren des Katalysators, Nachwaschen mit 20 ml Wasser und Ansäuern mit 50 %iger Schwefelsäure auf pH 3,25 bei 20°C wird mit 4 x 50 ml Methylisobutylketon extrahiert, Unter Absenken des Druckes bis auf 5 mbar wird dann bei Temperaturen von ≦ 60°C das Lösungsmittel abgezogen. Es verbleiben 14,1 g eines Rückstandes, der gemäß Gaschromatogramm zu 97,0 % aus 2,2,5-Trimethyl-1,3-dioxan-5-carbonsäure besteht. Die Ausbeute errechnet sich danach zu 78,5 % der Theorie. Fp. 119-121°C
Nach Umkristallisation aus Ligroin: Fp. 121 bis 123°C

Als Verunreinigungen sind 0,8 % Methylisobutylketon, 1,7 % Ausgangsmaterial und 0,5 % andere Verbindungen enthalten.

Beispiel 5

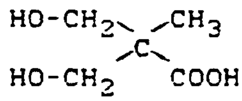

34,8 g (0,2 Mol) 2,2,5-Trimethyl-1,3-dioxan-carbonsäure und 100 g Wasser werden unter Normaldruck auf 95 bis 102°C erhitzt. Das dabei entstehende Aceton wird über eine 30 cm x 1,5 cm Silberkolonne mit 4 mm VA-Netzen bei einem Rücklaufverhältnis von 10:1 mit konstanter Geschwindigkeit abdestilliert. Die Kopftemperatur bleibt etwa 30 Minuten konstant bei 56°C und steigt dann innerhalb von 10 Minuten auf ca. 100°C an. Die in dieser Zeit (40 Minuten) erhaltene Destillatmenge beträgt 11,5 g und enthält laut gaschromatographischer Analyse 10,2 g Aceton und 1,3 g Wasser. Die als Sumpfprodukt verbleibende farblose wäßrige Lösung wird bei 60°C unter Absenken des Druckes bis auf 2 mbar zur Trockne eingedampft. Das dabei anfallende Destillatwasser enthält weitere 0,7 g Aceton. Als Rückstand verbleiben 25,8 g eines Produktes, das gemäß Gaschromatogramm zu 99,5 % aus Dihydroxypivalinsäure besteht. Fp. 189 bis 191°C.

Die Ausbeute errechnet sich zu 95,6 % der Theorie.
Aceton-Ausbeute: 93,8 % der Theorie.

Beispiel 6

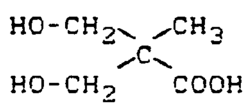

Eine Lösung von 16 g (0,1 mol) 2,2,5-Trimethyl-5-hydroxymethyl-1,3-dioxan in 100 ml (0,12 Mol) 1,1 n wäßriger Natronlauge wird nach Zugabe von 1 g 5 % Palladium enthaltender Aktivkohle mit 0,030 g $Bi(NO_3)_3 \cdot 5\ H_2O$ versetzt. Danach wird bei 80°C in der im Beispiel 4 beschriebenen Weise mit 0,1 Mol Sauerstoff oxidiert.

Nach dem Absaugen des Katalysators, Nachwaschen mit Wasser und Ansäuern mit 20 %iger Salzsäure auf pH 1,5 werden Aceton und Wasser abdestilliert. Der trockene, salzhaltige Rückstand wird mit warmem Ethanol extrahiert. Nach Abdampfen des Ethanols vom Extrakt verbleiben 14,3 g fester, weißer Rückstand, der nach dem Gaschromatogramm 13,2 g (98,5 % der Theorie) 2,2-Bishydroxymethylpropionsäure (Dihydroxypivalinsäure) enthält.

Beispiel 7

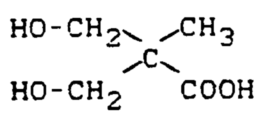

Es wird wie in Beispiel 6 gearbeitet, jedoch ohne Zusatz einer Bismut-Verbindung.

Nach 120 Minuten sind 0,1 Mol Sauerstoff aufgenommen und die Oxidation ist beendet. Die Aufarbeitung in der im Beispiel 6 beschriebenen Weise ergibt 10,2 g Rückstand, der nach dem Gaschromatogramm 9,5 g (70,9 % der Theorie) 2,2-Bishydroxymethyl-propionsäure enthält.

Beispiel 8 bis 10

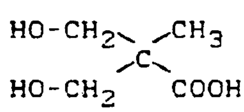

Es wird wie in Beispiel 6 gearbeitet, jedoch mit dem Unterschied, daß an Stelle des Palladium/Kohle-Katalysators 1 g Aktivkohle mit 1 Gew.-% Platingehalt in Abwesenheit (Vergleichsbeispiel 8) oder Anwesenheit von 30 mg $Bi(NO_3)_3 \cdot 5H_2O$ (Beispiel 9) oder 0,5 ml 0,5 m $Pb(NO_3)_2$-Lösung (Beispiel 10) als Aktivator verwendet wird. Es werden die in der folgenden Tabelle 1 aufgeführten Ergebnisse erzielt:

Tabelle 1

Einfluß von Bismut und Blei auf die Oxidation von 2,2,5-Trimethyl-5-hydroxymethyl-1,3-dioxan (16 g ≙ 0,1 Mol) in 1,1 n wäßriger Natronlauge (100 ml) bei 80° C unter Normaldruck an Aktivkohle (1 g) mit 1 Gew.-% Platingehalt.

| Beispiel | Aktivator | $O_2$-Aufnahme | | Ausbeute % der Theorie |
|---|---|---|---|---|
| | | $\frac{\text{Mol } O_2}{\text{Mol Dioxan}}$ | erforderl. Zeit [h] | |
| 8+) | ohne | 0,025 | 4 | < 2 |
| 9 | $Bi(NO_3)_3$ | 1,00 | 2,25 | 82,9 |
| 10 | $Pb(NO_3)_2$ | 1,00 | 2,0 | 89,8 |

+) zum Vergleich

Die Beispiele 9 bis 10 zeigen die starke Wirkung der erfindungsgemäß verwendbaren Aktivatoren.

Beispiel 11

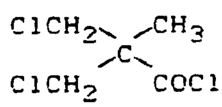

Zu 32 g (0,2 Mol) 2,2,5-Trimethyl-5-hydroxymethyl-1,3-dioxan werden 100 ml (0,22 Mol) wäßrige Natronlauge und 1 g Aktivkohlepulver mit 5 Gew.-% Palladiumgehalt sowie 30 mg $Bi(NO_3)_3 \cdot 5\ H_2O$ gegeben.

Nach dem Verdrängen der Luft aus dem Reaktionsgefäß durch Sauerstoff und Erwärmen auf 80°C wird unter kräftiger Durchmischung Sauerstoff unter Normaldruck eingeleitet bis die Sauerstoff-Aufnahme nach etwa 5 Stunden zum Stillstand kommt und 0,2 Mol Sauerstoff aufgenommen sind.

Nach dem Abfiltrieren des Katalysators (er kann wiederverwendet werden) wird das Filtrat mit 20 %iger Salzsäure auf pH 2 angesäuert und zur Trockne eingedampft. Als Destillat wird ein Aceton-Wasser-Gemisch erhalten. Als fester Rückstand verbleiben 39,7 g Dihydroxypivalinsäure-Natriumchlorid-Gemisch, das mit 119 g (1 Mol) Thionylchlorid und 0,5 g Dimethylformamid langsam - nach Maßgabe der $HCl/SO_2$-Entwicklung - im Verlauf von 2 Stunden auf Rückflußtemperatur erhitzt und dann 6 Stunden bei dieser Temperatur gerührt wird. Die Sumpftemperatur beträgt zum Schluß etwa 100°C.

Dann wird über eine Destillationsbrücke zunächst das überschüssige Thionylchlorid abdestilliert und dann bei 18 bis 20 mb/84 bis 108°C Kopftemperatur das ß,ß'-Dichlorpivalinsäurechlorid. Von letzterem erhält man 35 g in 96 %iger Reinheit (= 88,7 % der Theorie), bezogen auf eingesetztes 2,2,5-Trimethyl-5-hydroxymethyl-1,3-dioxan.

Aus dem Sumpf lassen sich mit Methylenchlorid 3,2 g organisches Material extrahieren, das laut Gaschromatogramm weitere 1 g (2,6 % der Theorie) ß,ß'-Dichlorpivalinsäurechlorid enthält. Der nicht in Methylenchlorid lösliche Anteil besteht aus 12,3 g Natriumchlorid und 0,2 g wasserunlöslichen Harzen.

Beispiel 12

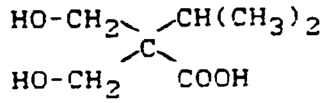

In die im Beispiel 1 beschriebene Apparatur werden 9,4 g (0,05 Mol) 2,2-Dimethyl-5-isopropyl-5-hydroxymethyl-1,3-dioxan (Öl, Kp. 106 bis 107°C/5 mb), 100 ml 1 n wäßrige Natronlauge, 1 g Aktivkohle mit 5 Gew.-% Palladiumgehalt und 0,030 g $Bi(NO_3)_3 \cdot 5\ H_2O$ eingebracht. Es wird wie im Beispiel 1 beschrieben bei 90°C oxidiert. Das zunächst nur unvollständig in der Natronlauge lösliche 2,2-Dimethyl-5-isopropyl-5-hydroxymethyl-1,3-dioxan geht im Verlauf der Oxidation unter Bildung des Natriumsalzes der betreffenden Carbonsäure in Lösung. Nachdem im Verlauf von 4 Stunden etwa 0,048 Mol Sauerstoff aufgenommen worden sind und die Sauerstoff-Aufnahme fast zum Stillstand gekommen ist, wird die Oxidation beendet.

Nach dem Abfiltrieren des Katalysators und Nachwaschen mit etwas Wasser werden aus dem wäßrig-alkalischen Filtrat mit Ether 0,3 g nicht umgesetztes Ausgangsmaterial extrahiert. Die wäßrig-alkalische Phase wird dann mit 20 %iger Salzsäure auf pH 1 angesäuert und zur Trockne eingedampft. Im abdestillierten Wasser sind gemäß gaschromatographischer Analyse etwa 2,5 g Aceton enthalten. Die mehrfache Extraktion des festen Abdampfrückstandes mit heißem Ethanol ergibt nach dem Abziehen des Ethanols von den vereinigten Extrakten 8,0 g festen Rückstand, der gemäß gaschromatographischer Analyse 7,8 g (96,3 % der Theorie) 2,2-Bis-hydroxymethyl-isovaleriansäure enthält. Fp. 146 bis 147°C nach Umlösung aus Butylacetat. Das NMR-Spektrum bestätigt die Struktur.

Beispiel 13

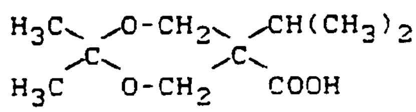

9,4 g (0,05 Mol) 2,2-Dimethyl-5-isopropyl-5-hydroxymethyl-1,3-dioxan werden wie im Beispiel 12 in 100 ml 1 n wäßriger Natronlauge in Gegenwart von 1 g Aktivkohle mit 5 % Palladiumgehalt und 0,030 g $Bi(NO_3)_3 \cdot 5\ H_2O$ bei 90°C 3,5 Stunden lang mit Sauerstoff unter Normaldruck oxidiert. Danach sind etwa 0,048 Mol Sauerstoff aufgenommen.

Nach dem Abfiltrieren des Katalysators und Nachwaschen mit Wasser werden aus dem wäßrig-alkalischen Filtrat mit Ether etwa 0,4 g nicht umgesetztes Ausgangsmaterial extrahiert. Dann wird die wäßrig-alkalische Phase mit 20 %iger Salzsäure auf pH 3,5 angesäuert und erneut mit Ether extrahiert. Nach Abziehen des Ethers von den vereinigten Extrakten verbleiben 7,2 g Feststoff (Fp. 78 bis 81°C), der gemäß gaschromatographischer Analyse 6,9 g (68,3 % der Theorie) 2,2-Dimethyl- 5-isopropyl-1,3-dioxan-5-carbonsäure und 0,25 g (3,1 % der Theorie) 2,2-Bis-hydroxymethylisovaleriansäure enthält. Weiteres Rohprodukt befindet sich in der wäßrigen Phase.

Der Fp. der 2,2-Dimethyl-5-isopropyl-1,3-dioxan-5-carbonsäure beträgt nach Umlösen aus Ligroin 81 bis 83°C. Die Struktur wird durch das NMR-Spektrum bestätigt.

Beispiel 14

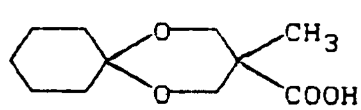

10,0 g (0,05 Mol) 3-Hydroxymethyl-3-methyl-1,5-dioxaspiro-[5,5]undecan werden wie im Beispiel 12 in 100 ml 1 n wäßriger Natronlauge suspendiert und in Gegenwart von 1 g Aktivkohle mit 5 % Palladiumgehalt und 0,030 g $Bi(NO_3)_3 \cdot 5\ H_2O$ bei 90°C 4 Stunden lang mit Sauerstoff unter Normaldurck oxidiert. Danach sind etwa 0,05 Mol Sauerstoff aufgenommen.

Nach dem Abfiltrieren des Katalysators und Nachwaschen mit Wasser werden aus dem wäßrig-alkalischen Filtrat mit Ether 1,3 g nicht umgesetztes Ausgangsmaterial extrahiert. Dann wird die wäßrig-alkalische Phase mit 20 %iger Salzsäure auf pH 3,5 angesäuert und erneut mit Ether extrahiert. Nach dem Abziehen des Ethers erhält man 9,1 g eines Produktes, das gemäß Gaschromatogramm zu 96 % aus 3-Carboxy-3-methyl-1,5-dioxa-spiro-[5,5]-undecan besteht.

Fp. 108 bis 110°C
(nach Umkristallisation aus Ligroin: Fp. 111°C)

Beispiel 15

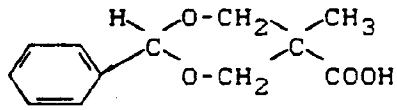

Zu 10,4 g eines Produktes, das zu 95 % aus 2-Phenyl-5-methyl-5-hydroxymethyl-1,3-dioxan-Isomerengemisch (0,047 Mol) besteht, werden 100 ml 1 m wäßrige Natronlauge, 1 g Aktivkohlepulver mit 5 Gew.-% Palladiumgehalt und 30 mg $Bi(NO_3)_3 \cdot 5\ H_2O$ gegeben. Dann wird nach der im Beispiel 1 beschriebenen Methode bei 90°C unter guter Durchmischung Sauerstoff in das Reaktionsgemisch eingeleitet. Nach 180 Minuten sind etwa 0,045 Mol Sauerstoff aufgenommen worden und die Sauerstoffaufnahme ist zum Stillstand gekommen. Nach dem Absaugen des Katalysators wird das alkalische Filtrat mit Ether extrahiert. Durch Eindampfen der organischen Phase werden 0,3 g Ausgangsmaterial zurückgewonnen. Die wäßrige Phase wird mit 20 %iger Salzsäure auf pH 3,5 angesäuert. Die dabei ausfallenden weißen Kristalle werden mit Ether aufgenommen, und die entstehende Lösung wird mehrfach mit Ether ausgeschüttelt. Die vereinigten organischen Phasen werden nach dem Trocknen über Natriumsulfat durch Abziehen des Lösungsmittels eingeengt. Man erhält 9,5 g eines Produktes, das gemäß Gaschromatogramm zu 95 % aus 2-Phenyl-5-methyl-1,3-dioxan-5-carbonsäure-Isomerengemisch besteht, wobei die Isomeren im Verhältnis 73:27 vorliegen. Die Ausbeute errechnet sich zu 85,6 % der Theorie.
Fp. 180 bis 183°C.

**Patentansprüche**

1. Verfahren zur Herstellung von Carbonsäure-Derivaten der Formel

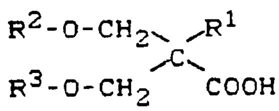

(I)

in welcher
$R^1$ für Wasserstoff, Alkyl, Cycloalkyl oder gegebenenfalls substituiertes Phenyl steht und
$R^2$ und $R^3$ für Wasserstoff stehen oder
$R^2$ und $R^3$ gemeinsam für die Gruppierung der Formel

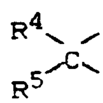

stehen, worin
$R^4$ und $R^5$ unabhängig voneinander für Wasserstoff, Alkyl, Cycloalkyl oder gegebenenfalls substituiertes Phenyl stehen oder
$R^4$ und $R^5$ gemeinsam für eine Alkylenkette stehen,
dadurch gekennzeichnet, daß man 5-Hydroxymethyl-1,3-dioxane der Formel

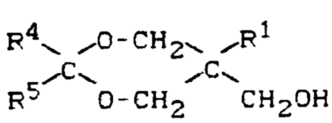

(II)

in welcher
$R^1$, $R^4$ und $R^5$ die oben angegebene Bedeutung haben,

in wäßrig-alkalischem Medium mit Saurerstoff oder Sauerstoff enthaltenden Gasen bei Temperaturen zwischen 0°C und dem Siedepunkt des Reaktionsgemisches an einem Palladium-und/oder Platin-Katalysator, gegebenenfalls in Gegenwart eines Aktivators umsetzt und die so erhaltenen Salze mit Kationenaustauschern oder Säuren behandelt und gegebenenfalls anschließend die dabei entstehenden 1,3-Dioxan-5-carbonsäuren der Formel

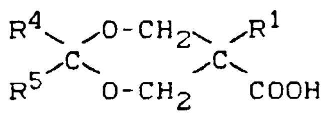

(Ia)

in welcher

$R^1$, $R^4$ und $R^5$ die oben angegebene Bedeutung haben,

oder deren Salze mit Wasser, gegebenenfalls in Gegenwart eines Katalysators sowie gegebenenfalls in Gegenwart eines zusätzlichen Verdünnungsmittels bei Temperaturen zwischen 0°C und dem Siedepunkt des Reaktionsgemisches umsetzt.

2. Verfahren gemäß Anspruch 1, dadurch gekennzeichnet, daß man 5-Hydroxymethyl-1,3-dioxane der Formel (11) einsetzt, in denen $R^1$, $R^4$ und $R^5$ unabhängig voneinander für Wasserstoff, geradkettiges oder verzweigtes Alkyl mit 1 bis 12 Kohlenstoffatomen, Cycloalkyl mit 3 bis 8 Kohlenstoffatomen oder für gegebenenfalls durch Halogen und/oder Alkyl mit 1 bis 6 Kohlenstoffatomen substituiertes Phenyl stehen oder $R^4$ und $R^5$ gemeinsam für eine Alkylenkette mit 4 bis 6 Kohlenstoffatomen stehen.

3. Verfahren gemäß Anspruch 1, dadurch gekennzeichnet, daß man Platin- und/oder Palladium-Katalysatoren in Form von Trägerkatalysatoren einsetzt.

4. Verfahren gemäß Anspruch 3, dadurch gekennzeichnet, daß man Aktivkohle als Katalysatorträger verwendet.

5. Verfahren gemäß Anspruch 1, dadurch gekennzeichnet, daß man die Umsetzung in Gegenwart eines Aktivators durchführt.

6. Verfahren gemäß Anspruch 5, dadurch gekennzeichnet, daß man Blei, Bismut, Bleiverbindungen, Bismutverbindungen und/oder deren Gemische als Aktivatoren einsetzt.

7. Verfahren gemäß Anspruch 1, dadurch gekennzeichnet, daß man die Umsetzung in Gegenwart wäßriger Lösungen von Alkalimetall- und/oder Erdalkalimetall-Verbindungen durchführt.

8. Verfahren gemäß Anspruch 1, dadurch gekennzeichnet, daß man als Sauerstoff enthaltendes Gas Luft einsetzt.

9. Verfahren gemäß Anspruch 1, dadurch gekennzeichne!, daß man bei Temperaturen zwischen 40°C und 100°C arbeitet.

## Claims

1. Process for the preparation of carboxylic acid derivatives of the formula

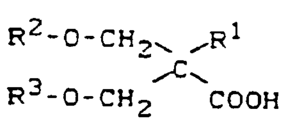

(I)

in which

$R^1$ represents hydrogen, alkyl, cycloalkyl or optionally substituted phenyl, and

$R^2$ and $R^3$ represent hydrogen, or

$R^2$ and $R^3$ together represent the group of the formula

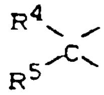

,

wherein

$R^4$ and $R^5$, independently of one another, represent hydrogen, alkyl, cycloalkyl or optionally substituted

phenyl, or

$R^4$ and $R^5$ together represent an alkylene chain,

characterised in that 5-hydroxymethyl-1,3-dioxanes of the formula

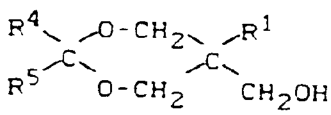

(II)

in which

$R^1$, $R^4$ and $R^5$ have the abovementioned meaning,

are reacted with oxygen or oxygen-containing gases in aqueous alkaline medium at temperatures between 0°C and the boiling point of the reaction mixture on a palladium and/or platinum catalyst, if appropriate in the presence of an activator, and the salts thus obtained are treated with cation exchangers or acids, and, if appropriate, the 1,3-dioxane-5-carboxylic acids of the formula

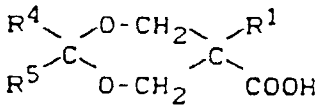

**(Ia)**

in which

$R^1$,$R^4$ and $R^5$ have the abovementioned meaning,

produced from this treatment, or their salts, are subsequently reacted with water, if appropriate in the presence of a catalyst and if appropriate in the presence of an additional diluent at temperatures between 0°C and the boiling point of the reaction mixture.

2. Process according to Claim 1, characterised in that 5-hydroxymethyl-1,3-dioxanes of the formula (II) are employed in which $R^1$, $R^4$ and $R^5$, independently of one another, represent hydrogen, straight-chain or branched alkyl having 1 to 12 carbon atoms, cycloalkyl having 3 to 8 carbon atoms, or phenyl which is optionally substituted by halogen and/or alkyl having 1 to 6 carbon atoms, or $R^4$ and $R^5$ together represent an alkylene chain having 4 to 6 carbon atoms.

3. Process according to Claim 1, characterised in that platinum and/or palladium catalysts in the form of supported catalysts are employed.

4. Process according to Claim 3, characterised in that activated charcoal is used as catalyst support.

5. Process according to Claim 1, characterised in that the reaction is carried out in the presence of an activator.

6. Process according to Claim 5, characterised in that lead, bismuth, lead compounds, bismuth compounds and/or their mixtures are employed as activators.

7. Process according to Claim 1, characterised in that the reaction is carried out in the presence of aqueous solutions of alkali metal and/or alkaline earth metal compounds.

8. Process according to Claim 1, characterised in that air is employed as oxygen-containing gas.

9. Process according to Claim 1, characterised in that the process is carried out at temperatures between 40°C and 100°C.

## Revendications

1. Procédé de préparation de dérivés d'acides carboxyliques de formule :

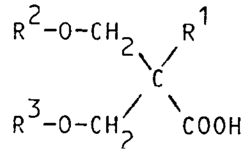

(I)

dans laquelle

$R^1$ représente l'hydrogène, un groupe alkyle, cycloalkyle ou un groupe phényle éventuellement substitué, et

$R^2$ et $R^3$ représentent l'hydrogène, ou bien

$R^2$ et $R^3$ forment ensemble le groupement de formule

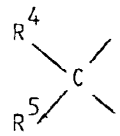

dans lequel

$R^4$ et $R^5$ représentent chacun, indépendamment l'un de l'autre, l'hydrogène, un groupe alkyle, cycloalkyle ou un groupe phényle éventuellement substitué, ou bien

$R^4$ et $R^5$ forment ensemble une chaîne alkylène,

caractérisé en ce que l'on fait réagir des 5-hydroxyméthyl-1,3-dioxannes de formule

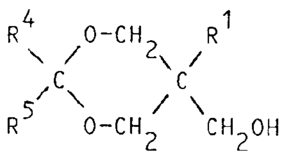

(II)

dans laquelle

$R^1$, $R^4$ et $R^5$ ont les significations indiquées ci-dessus,

en milieu aqueux alcalin, avec l'oxygène ou des gaz contenant de l'oxygène, à des températures allant de 0°C jusqu'au point d'ébullition du mélange de réaction, sur un catalyseur au palladium et/ou au platine, éventuellement en présence d'un activateur, et on traite les sels ainsi obtenus par des échangeurs de cations ou des acides, et le cas échéant on fait ensuite réagir les acides 1,3-dioxanne-5-carboxyliques ainsi obtenus, de formule

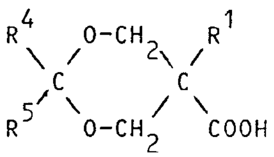

(Ia)

dans laquelle

$R^1$, $R^4$ et $R^5$ ont les significations indiquées ci-dessus,

ou leurs sels, avec de l'eau, éventuellement en présence d'un catalyseur et éventuellement en présence d'un diluant complémentaire, à des températures allant de 0°C jusqu'au point d'ébullition du mélange de réaction.

2. Procédé selon la revendication 1, caractérisé en ce que l'on met en oeuvre des 5-hydroxyméthyl-1,3-dioxannes de formule II dans laquelle $R^1$, $R^4$ et $R^5$ représentent chacun, indépendamment les uns des autres, l'hydrogène, un groupe alkyle à chaîne droite ou ramifiée en $C_1$-$C_{12}$, un groupe cycloalkyle en $C_3$-$C_8$ ou un groupe phényle éventuellement substitué par des halogènes et/ou des groupes alkyle en $C_1$-$C_6$, ou bien $R^4$ et $R^5$ forment ensemble une chaîne alkylène en $C_4$-$C_6$.

3. Procédé selon la revendication 1, caractérisé en ce que l'on met en oeuvre les catalyseurs au platine et/ou au palladium à l'état de catalyseurs sur supports.

4. Procédé selon la revendication 3, caractérisé en ce que l'on utilise le charbon actif en tant que support de catalyseur.

5. Procédé selon la revendication 1, caractérisé en ce que l'on effectue la réaction en présence d'un activateur.

6. Procédé selon la revendication 5, caractérisé en ce que l'on utilise en tant qu'activateurs le plomb, le bismuth, des composés du plomb, des composés du bismuth et/ou leurs mélanges.

7. Procédé selon la revendication 1, caractérisé en ce que l'on effectue la réaction en présence de solutions aqueuses de composés de métaux alcalins et/ou alcalino-terreux.

8. Procédé selon la revendication 1, caractérisé en ce que l'on utilise l'air en tant que gaz contenant de l'oxygène.

9. Procédé selon la revendication 1, caractérisé en ce que l'on opère à des températures de 40 à 100°C.